Europäisches Patentamt

**European Patent Office**

Office européen des brevets

(11) Numéro de publication: **0 245 160**
**B1**

(12) **FASCICULE DE BREVET EUROPEEN**

(45) Date de publication du fascicule du brevet: **30.05.90**

(21) Numéro de dépôt: **87400987.1**

(22) Date de dépôt: **29.04.87**

(51) Int. Cl.⁵: **H 05 G 1/08, H 05 G 1/30, H 05 G 1/70, H 04 B 10/00**

(54) **Installation de radiologie à réseau de communication.**

(30) Priorité: **06.05.86 FR 8606546**

(43) Date de publication de la demande:
**11.11.87 Bulletin 87/46**

(45) Mention de la délivrance du brevet:
**30.05.90 Bulletin 90/22**

(84) Etats contractants désignés:
**DE FR NL**

(56) Documents cités:
**EP-A-0 066 928**
**EP-A-0 136 645**
**FR-A-2 115 876**
**FR-A-2 247 871**
**FR-A-2 354 017**
**US-A-4 533 947**

**GLOBECOM '85 IEEE GLOBAL
TELECOMMUNICATIONS CONFERENCE,
Conference Record, 2-5 décembre 1985, vol. 3,
pages 1185-1189, IEEE Communications
Society, New Orleans, Louisiana, US; H. KELLER
et al.: "Overlay optical-fiber local-area network"**

(73) Titulaire: **GENERAL ELECTRIC CGR S.A.
100, rue Camille-Desmoulins
F-92130 Issy les Moulineaux (FR)**

(72) Inventeur: **Audon, Philippe
THOMSON-CSF SCPI 19, avenue de Messine
F-75008 Paris (FR)**
Inventeur: **Courtecuisse, Daniel
THOMSON-CSF SCPI 19, avenue de Messine
F-75008 Paris (FR)**

(74) Mandataire: **Ballot, Paul Denis Jacques
Cabinet Ballot-Schmit 7, rue le Sueur
F-75116 Paris (FR)**

Courier Press, Leamington Spa, England.

EP 0 245 160 B1

**Description**

L'invention est relative à une installation de radiologie.

Une installation de radiologie, notamment radioscopie ou radiographie, comporte un nombre important d'appareils qui sont associés les uns aux autres en fonction des besoins. Par exemple, pour effectuer une radioscopie on fait appel à un générateur de rayons X, un intensificateur d'images, une caméra vidéo et un récepteur ou moniteur de télévision; pour la radiographie, à la place d'une caméra vidéo et d'un moniteur de télévision on utilise un appareil photographique à changeur de films. Lors de l'exécution d'un examen d'un type déterminé un certain nombre d'appareils sont mis en service, les autres étant hors d'action, et des connexions particulières sont établies entre les appareils en service. Ainsi pour chaque utilisation on confère à l'installation une configuration déterminée. Pour permettre la modification instantanée de la configuration on prévoit souvent un câblage complexe de l'installation.

Pour simplifier le montage on a déjà proposé une installation de radiologie dans laquelle le générateur de rayons X est associé à d'autres appareils suivant une boucle fermée où les informations sont transmises d'un appareil à un autre par l'intermédiaire d'une fibre optique. Quand un appareil n'est pas utilisé ou est en panne on dérive l'information par un commutateur de façon à maintenir la boucle fermée.

Cette installation connue présente des limitations qui peuvent réduire à néant l'intérêt présenté par la simplification du câblage. On a constaté en particulier que lorsqu'on a besoin de transmettre un grand débit d'informations entre deux appareils la ligne que constitue la boucle fermée peut être saturée et empêche simultanément un grand débit d'informations entre d'autres appareils.

L'invention remédie à ces inconvénients.

Elle est caractérisée par les caractéristiques de la deuxième partie de la revendication 1.

Avec une telle installation les informations peuvent circuler simultanément à grand débit sur deux boucles distinctes et il est possible de faire communiquer deux appareils se trouvant sur des boucles différentes.

Par exemple un traitement d'images enregistré dans un appareil de traitement numérique peut nécessiter un échange d'informations à fort débit entre un pupitre de commande et cet appareil; il en est ainsi notamment quand on utilise un instrument de dialogue du type écran tactile "boule roulante" ou "joy-stick". Avec l'invention si les commandes de l'appareil photographique, de l'intensificateur d'images et du collimateur d'images se trouvent dans une boucle distincte de celle où se trouve l'appareil de traitement numérique il est possible d'effectuer une radiographie en même temps qu'on exécute un traitement d'image.

Une boucle secondaire comporte par exemple les commandes de position des appareils de la salle de radiologie, à savoir la table porte-patient et le support, en général en forme d'arceau, de l'ensemble tube à rayons X-détecteurs. Une autre boucle secondaire ou spécialisée est affectée à la commande des appareils de formation d'image tel qu'un appareil photographique et une caméra vidéo.

Pour une installation selon l'invention, on a trouvé que l'utilisation du protocole de communication de type HDLC (High Level data link control) était particulièrement avantageuse.

Pour réaliser l'installation de l'invention on affecte à chaque appareil un dispositif de communication et de traitement qui comporte de façon séparée un circuit de gestion des communications avec les autres appareils et un circuit de traitement des messages destinés à l'appareil ou provenant de l'appareil.

Le circuit de gestion des communications est par exemple tel qu'il comporte un commutateur constitué par un seul interrupteur en dérivation qui se ferme lors de la détection d'un incident dans l'appareil correspondant.

Les signaux dans chaque boucle sont du type électrique ou optique.

Dans le mode de réalisation préféré de l'invention, on prévoit dans la boucle, principale, un organe central de gestion qui, à chaque mise sous tension de l'installation, détermine la configuration de cette dernière, c'est-à-dire détermine dans quelle boucle se trouve chaque appareil et garde en mémoire cette configuration, cette détermination étant effectuée à l'aide d'organes annexes de gestion qui se trouvent aux jonctions entre les boucle voisines et qui transmettent vers l'organe central de gestion la liste des appareils connectés à la boucle la plus éloignée de la boucle principale.

Avec une telle installation, le trajet de communication entre deux appareils est déterminé automatiquement à chaque utilisation sans qu'il soit fonction de la connexion. En effet, quand un premier appareil sur une première boucle doit communiquer avec un second appareil sur une seconde boucle, distincte de la première, on fait émettre au premier appareil un message sur sa boucle et ce message est reçu par l'organe annexe de gestion de cette boucle qui garde en mémoire la configuration de la première boucle et de la boucle voisine et qui oriente ainsi le message vers cette boucle voisine. Si le second appareil ne se trouve pas sur cette boucle voisine, le message est transmis par d'autres organes annexes de gestion aux noeuds entre cette boucle voisine et une autre boucle, et ainsi de suite jusqu'à ce que soit atteint la seconde boucle et le second appareil.

En outre, avec un dispositif de communication et de traitement affecté à chaque appareil ainsi qu'avec un organe central de gestion et des organes annexes de gestion à chaque noeud, le dispositif de communication et de traitement de chaque appareil peut être de nature identique pour tous les appareils, c'est-à-dire être indépendant du type d'appareil et de la configuration de l'installation, ce qui permet, en plus d'une réduction des coûts, de modifier la configuration par de simples, modifications des connexions entre appareils, sans autres modifications, car, comme indiqué ci-dessus, la configuration est déterminée à chaque mise sous tension et le trajet de communication entre appareil est établi de façon automatique.

D'autres caractéristiques et avantages de l'invention apparaîtront avec la description de certains de ses modes de réalisation, celle-ci étant effectuée en se référant aux dessins ci-annexés sur lesquels:

la figure 1 est un schéma d'une installation selon l'invention,

la figure 2 est un schéma d'un dispositif de communication associé à chaque appareil de l'installation de la figure 1,

la figure 3 montre une particularité du dispositif de la figure 2, et

la figure 4 est un schéma simplifié correspondant à la figure 1 qui permet de mieux comprendre le fonctionnement de l'installation.

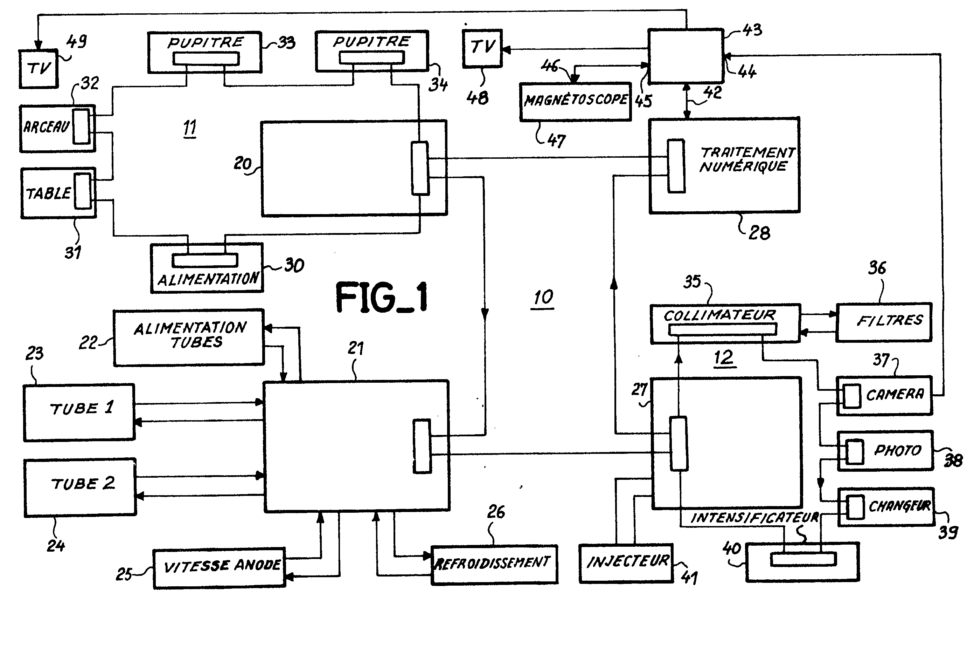

L'installation de radiologie représentée sur la figure 1 permet d'effectuer des examens radioscopiques et radiographiques ainsi que des traitements numériques d'images.

Les messages (informations de commande ou d'état) sont transmis par l'intermédiaire de lignes de communication constituées par des fibres optiques et organisées suivant plusieurs boucles avec une boucle principale 10 et deux boucles secondaires 11 et 12.

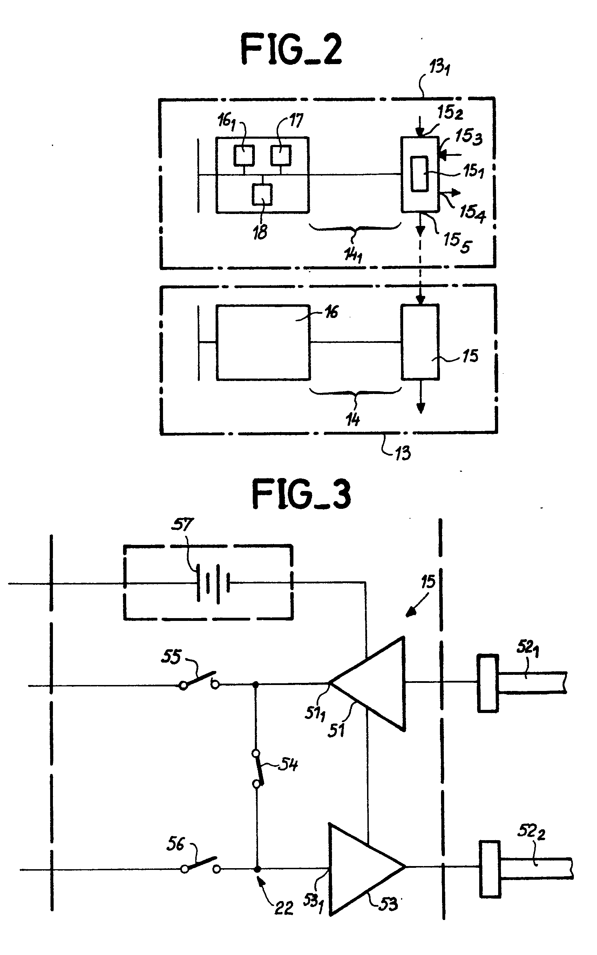

Comme on peut le voir sur la figure 2, à chaque appareil 13 est associé un dispositif de communication 14 comprenant, d'une part, un circuit 15 de gestion de la communication des signaux entre appareils et, d'autre part, un circuit 16 de traitement des informations destinées à—ou provenant de—l'appareil 13 proprement dit. Chaque circuit comporte un moyen de calcul à microprocesseur. Pour le circuit 15 de gestion des communications l'unité centrale du microprocesseur porte la référence $15_1$ tandis que pour le circuit 16 l'unité centrale du microprocesseur porte la référence $16_1$. Ce circuit 16 comporte en outre une mémoire 17 et un circuit 18 de gestion des entrées/sorties parallèles.

Les appareils se trouvant à un noeud, comme celui de référence $13_1$ sur la figure 2, qui sont entre la boucle principale 10 et une boucle secondaire 11 ou 12, sont associés à des dispositifs de communication $14_1$ qui comportent un circuit de gestion de communications à deux entrées $15_2$ et $15_3$ et deux sorties $15_4$ et $15_5$.

Les appareils dont les commandes se trouvent sur la boucle principale 10 sont:

un dispositif 20 de commande et contrôle de salle se trouvant également dans la boucle secondaire 11 dite boucle de salle,

un dispositif 21 de contrôle et commande de la puissance du rayonnement X qui assure la commande et le contrôle d'une armoire de puissance 22 pour l'alimentation en énergie électrique de deux tubes 23 et 24 à rayons X, d'un dispositif 25 de commande de vitesse de rotation de l'anode tournante de chaque tube 23, 24, et d'un dispositif de refroidissement 26,

un dispositif 27 de contrôle d'images se trouvant également dans la boucle secondaire 12 dite boucle d'image,

et un dispositif 28 de traitement numérique d'images.

Dans la boucle secondaire 11 de salle se trouve, à la suite du dispositif 20, un dispositif 30 de commande de l'alimentation en énergie électrique des appareils de la salle de radiologie et de l'éclairage de cette salle, les commandes 31 de position de la table porte-patient, les commandes 32 de position d'un arceau portant les tubes à rayons X et le (ou les) détecteur(s), un pupitre 33 se trouvant dans la salle de radiologie à proximité de la table porte-patient, et un pupitre 34 de commande générale.

Dans la boucle secondaire d'image 12 se trouve, à la suite du dispositif de contrôle d'images 27, la commande 35 d'un collimateur associé à des filtres de contour 36, la commande 37 d'une caméra vidéo, la commande 38 d'un appareil photographique, la commande 39 d'un changeur de films et la commande 40 d'un intensificateur d'images. En outre un dispositif 41 d'injection automatique de produit de contraste dans les vaisseaux sanguins du patient est commandé directement par le dispositif 27.

Le dispositif 28 de traitement numérique permet de réaliser de l'imagerie dite "soustractive" qui consiste à effectuer point par point la différence entre les valeurs de luminance affectées à chaque point, d'une part avec produit de contraste, et d'autre part sans produit de contraste. Ce procédé d'imagerie est surtout utilisé pour l'examen, appelé angiographie, des vaisseaux sanguins.

Le dispositif 28 est relié, par une ligne 42, à un circuit de distribution vidéo 43 présentant une entrée 44 recevant le signal de la caméra 37 et une entrée-sortie 45 reliée à une entrée-sortie 46 d'un magnétoscope 47. Le circuit 43 comporte, en outre, deux sorties reliées à deux moniteurs de télévision, respectivement 48 et 49, le premier étant disposé au voisinage du pupitre 34 de commande centrale et le second au voisinage de la table porte-patient.

Dans la boucle principale 10 et dans les boucles secondaires 11 et 12 les informations sont transmises d'un appareil à un autre par des fibres optiques. Ainsi à l'entrée du circuit 15 (figure 3) on prévoit un convertisseur 51 de signal lumineux, transmis par une fibre optique $52_1$, en un signal électrique et, à la sortie de ce circuit 15, se trouve un convertisseur 53 transformant un signal électrique provenant de ce circuit, ou acheminé par ce circuit, en un signal lumineux vers une fibre optique de sortie $52_2$.

La sortie $51_1$ du convertisseur d'entrée 51 est reliée directement à l'entrée $53_1$ du convertisseur de sortie 53 par l'intermédiaire d'un interrupteur 54 qui, en fonctionnement normal, est ouvert mais est fermé en cas d'incident dans l'appareil correspondant afin que la boucle correspondante reste fermée malgré l'incident. L'interrupteur 54 peut également intervenir quand l'appareil correspondant n'est pas utilisé.

En série avec le conducteur de sortie du convertisseur 51 se trouve un autre interrupteur 55. De même, en série avec le conducteur d'entrée 53 on prévoit un interrupteur 56. Les interrupteurs 55 et 56 sont fermés en fonctionnement normal et ouverts en cas d'incident.

Une pile électrique 57 alimente en énergie électrique les convertisseurs 51 et 53 afin que la transmission du signal puisse s'effectuer même en cas d'interruption de l'alimentation habituelle en énergie électrique de l'appareil correspondant 13.

On comprendra mieux les caractéristiques de l'installation de la figure 1 à l'aide de la description d'un exemple d'utilisation de cette installation. Cet exemple concerne la radiographie, c'est-à-dire l'utilisation de l'appareil photographique 38.

L'opérateur donne, grâce à un bouton sur le pupitre 34, un ordre d'examen radiographique. Cet ordre est transformé en un message acheminé par les circuits 16 et 15 associés à ce pupitre, vers le dispositif 20 de contrôle de salle qui, à partir de ce message reçu, élabore un autre message déterminant le trajet que doivent suivre les messages et qui est utilisé comme ordre de commande ou d'arrêt par les divers appareils de l'installation. Ainsi dans la boucle secondaire 11 le message provenant du pupitre 34 est interprété par la commande 30 comme étant un order de mise en route ou de maintien de l'alimentation en énergie électrique dans la salle. Cependant ce sont les messages préalablement envoyés grâce au pupitre 33 qui ont mis en place, dans la position voulue, la table 31 et l'arceau 32 supportant la source de rayons X et le (ou les) détecteur(s).

Le message du dispositif 20 est transmis dans la boucle principale 10 d'abord au dispositif 21 qui élabore une configuration d'utilisation des sources de rayons X et, ensuite, au dispositif 27 de contrôle d'images qui, dans la boucle secondaire 12, élabore aussi une configuration d'utilisation.

Le dispositif 21 détermine lesquels des deux tubes, 23 ou 24, doit être utilisé. Par exemple, si le tube 23 à fonctionné peu de temps auparavant on utilise seulement le tube 24. La vitesse de rotation de l'anode tournante est déterminée à la valeur correspondant à la radiographie, c'est-à-dire dans l'exemple 9.000 tours par minute. Le cas échéant le dispositif de refroidissement 26 est mis en route et les moyens d'alimentation 22 des tubes dont mis en veille en attendant l'ordre définitif de déclenchement de la prise de vue.

Dans la boucle secondaire 12 tous les appareils, à l'exception de la caméra de télévision, sont mis en état de veille.

Le message est retransmis au dispositif 20 par l'intermédiaire du dispositif 28 de traitement numérique d'image qui, dans ce cas, n'est pas utilisé.

Le message retransmis au dispositif 20 indique si le fonctionnement est correct ou non. En cas de réponse positive une indication est fournie sur le pupitre 34 et l'opérateur peut donner alors l'ordre définitif de prise de vue. En variante, une indication d'interdiction est fournie à l'opérateur en cas d'incident, une absence d'interdiction valant autorisation de prise de vue.

La configuration en boucle principale et boucles secondaires offre des possibilités supérieures à celles qu'on pourrait obtenir si toutes les informations transitaient par une boucle unique. Par exemple, les informations dans la boucle 12 ne passent pas par la boucle principale 10 et ne risquent donc pas de saturer le débit d'informations dans cette boucle principale. Réciproquement, les informations dans la boucle 12 peuvent circuler sans retard malgré une quantité importante d'informations dans la boucle 10. De cette manière, il est possible par exemple d'effectuer une radiographie alors qu'en même temps, on effectue un traitement d'images, à l'aide du pupitre 34 et des dispositifs 28 et 43, sur une radioscopie effectuée antérieurement. En effet un tel traitement d'images peut entraîner un grand débit d'informations sur la boucle 10 si on utilise, sur le pupitre, un écran tactile, une boule roulante ou un "joy-stick" pour effectuer des tracés sur l'image.

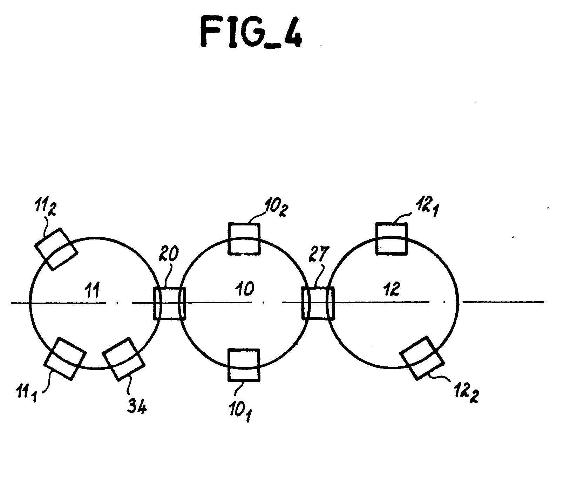

Sur la figure 4 on a représenté de façon simplifiée les trois boucles 10, 11, 12 avec le dispositif 20 connectant les boucles 10 et 11 et le dispositif 27 connectant les boucles 10 et 12. Dans la boucle 11 se trouvent le pupitre central de commande 34 et les appareils $11_1$, $11_2$ etc.. Dans la boucle 10 se trouvent les appareils $10_1$, $10_2$, etc.. Et dans la boucle 12 on a repéré par $12_1$, $12_2$, ... les appareils qui s'y trouvent. Cette représentation simplifiée permettra de mieux comprendre d'autres particularités de l'installation et de son fonctionnement.

A la mise sous tension de l'installation, à chaque utilisation, le dispositif 20 constitue l'organe central de gestion qui détermine la configuration de cette installation. A cet effet, ce dispositif 20 envoie un message dans la boucle 11 et chaque dispositif de gestion et communication associé à chaque appareil qui reçoit un message, renvoie, à ce dispositif 20, un message indiquant que le pupitre 34 et les appareils $11_1$ et $11_2$ se trouvent sur cette boucle 11. Le dispositif 20 garde ces informations en mémoire. Il envoie également un message dans la boucle 10. Les appareils $10_1$ et $10_2$ ainsi que le dispositif 27 indiquent qu'ils se trouvent sur la boucle 10 et cette information est gardée en mémoire dans le dispositif 20. Le dispositif 27 transmet le message de demande de configuration à la boucle 12 qui indique la présence des appareils $12_1$, $12_2$ et cette information sur les appareils $12_1$ et $12_2$ dans la boucle 12 est retransmise, par le même dispositif 27, au dispositif central 20. A partir de cette information, le dispositif 20 peut déterminer le trajet optimum d'un appareil à un autre. Par exemple pour la communication entre les appareils $11_1$ et $12_1$, l'information devra passer par les dispositifs 20 et 27.

En outre il est possible d'affecter le rôle principal de commande de l'installation au dispositif 27 au moins pour certaines opérations. Par exemple pour la préparation d'un examen radiologique le dispositif 20 constitue, comme décrit, l'organe central de commande; par contre, lors de l'acquisition d'images, c'est le dispositif 27 qui constitue l'organe central de commande, c'est-à-dire que ses messages sont prioritaires; en d'autres termes, un ordre donné par le dispositif 27 quand il constitue un organe central de commande l'emporte sur un ordre donné par le dispositif 20. Bien entendu, cet échange de rôles n'est pas effectué par une modification matérielle de l'installation mais par la programmation préalable des dispositifs de contrôle et commande.

La configuration de l'installation peut être modifiée aisément. Par exemple, si une communication doit être établie en permanence entre les appareils $11_1$ et $12_1$ on peut les disposer dans la même boucle. On modifie alors les connexions, par exemple en transportant l'appareil $12_1$ dans la boucle 11, sans qu'il soit besoin d'effectuer d'autres modifications. En effet, la configuration étant vérifiée périodiquement, elle est automatiquement prise en compte par les divers organes de contrôle et commande.

Il est à noter que cette configuration est non seulement déterminée à chaque mise sous tension mais également à chaque défaut ou incident détecté sur un appareil.

**Revendications**

1. Installation de radiologie comprenant un ensemble d'appareils entre lesquels circulent des signaux d'informations, caractérisée en ce que, pour la transmission de ces signaux, l'installation comporte au moins deux boucles (10, 11 et 12) entre lesquelles les informations peuvent circuler, chaque boucle étant susceptible de transmettre des informations qui ne passent pas par une autre boucle, chaque boucle reliant des appareils différents de l'installation, cette installation comportant une boucle principale (10) et des boucles secondaires ou spécialisées (11, 12), chaque boucle secondaire comportant un dispositif de commande et de contrôle (20, 27) pour l'ensemble des appareils de cette boucle, ce dispositif de contrôle et commande se trouvant au noeud entre cette boucle secondaire et la boucle principale (10), chaque appareil étant affecté d'un dispositif (14) de communication comprenant un circuit (16) de traitement de l'information pour l'appareil et un circuit (15) réservé à la transmission des messages.

2. Installation selon la revendication 1 caractérisée en ce qu'un pupitre de commande générale (34) est disposé dans l'une des boucles (11).

3. Installation selon la revendication 1 ou 2 caractérisée en ce que dans une première boucle secondaire (11) sont disposés les commandes (31, 32) de position des appareils de la salle de radiologie, à savoir la table porte-patient et le support de l'ensemble tube à rayons X-détecteur(s).

4. Installation selon la revendication 3 caractérisée en ce que dans la première boucle secondaire (11), en série avec les commandes de position, se trouvent la commande (30) de distribution d'énergie électrique aux appareils de la salle de radiologie et un pupitre (33) de commande de positions.

5. Installation selon la revendication 2 et 3 caractérisée en ce que le pupitre (34) de commande générale de l'installation est en série avec les commandes de positions (31, 32) des appareils dans la première boucle secondaire (11).

6. Installation selon l'une quelconque des revendications 1 à 5 caractérisée en ce que les commandes des appareils de formation d'images: appareil photographique (38) et caméra vidéo (37) sont disposées en série dans une deuxième boucle secondaire (12).

7. Installation selon la revendication 6 caractérisée en ce qu'au moins la commande (35, 39, 40) de l'un des éléments suivants est également disposée en série dans la deuxième boucle (12): un collimateur, un changeur de films et un intensificateur d'images.

8. Installation selon les revendications 1 et 6 caractérisée en ce que la commande (4) d'un dispositif d'injection atuomatique de produit de contraste est effectuée par le dispositif (27) de contrôle et commande au noeud entre la boucle principale (10) et la deuxième boucle secondaire (12).

9. Installation selon l'une quelconque des revendications 1 à 8 caractérisée en ce que dans la boucle principale (10) se trouve un dispositif (21) de contrôle des moyens générateurs de rayons X.

10. Installation selon l'une quelconque des revendications 1 à 9 caractérisée en ce que dans la boucle principale (10) se trouve un dispositif de calcul (28) pour effectuer un traitement numérique sur une image enregistrée.

11. Installation selon la revendication 1 caractérisée en ce que le circuit (15) de transmission des messages comporte un simple interrupteur (54) qui est fermé en cas d'incident de l'appareil correspondant afin de maintenir fermée la boucle dans laquelle se trouve cet appareil.

12. Installation selon l'une quelconque des revendications précédentes caractérisée en ce que les informations sont transmises par l'intermédiaire de fibres optiques (52), chaque circuit de transmission (15) comportant à son entrée un convertisseur (51) de signal lumineux en signal électrique et à sa sortie un convertisseur (53) de signal électrique en signal lumineux.

13. Installation selon l'une quelconque des revendications précédentes, caractérisée en ce qu'à chaque appareil est associé un dispositif de communication et de traitement, les divers dispositifs de communication et de traitement étant tous identiques.

14. Installation selon l'une quelconque des revendications précédentes, caractérisée en ce qu'elle comporte un organe central de gestion qui, à chaque mise sous tension de l'installation, d'une part détermine la configuration de cette dernière, c'est-à-dire identifie les appareils de l'installation et la boucle dans laquelle se trouve chacun des appareils, et d'autre part garde en mémoire cette information de configuration, la détermination étant effectuée par l'intermédiaire d'organes annexes de gestion aux connexions entre boucles voisines.

15. Installation selon la revendication 14, caractérisée en ce que pour certaines opérations, un organe de gestion constitue l'organe central, alors que pour d'autres opérations, un autre organe constitue cet organe central.

**Patentansprüche**

1. Röntgenanlage mit einem Satz von Geräten, zwischen denen Informationssignale verkehren, dadurch gekennzeichnet, daß für die Übermittlung dieser Signale die Anlage mindestens zwei Schleifen (10, 11 und 12) umfaßt, zwischen denen die Informationen verkehren können, wobei jede Schleife dazu fähig ist, Informationen zu übermitteln, die durch keine andere Schleife führen, wobei jede Schleife verschiedene Geräte der Anlage verbindet und diese Anlage eine Hauptschleife (10) und Nebenschleifen oder spezialisierte Schleifen (11, 12) umfaßt, wobei jede Nebenschleife eine Steuer- und Kontrollvorrichtung (20, 27) für die gesamten Geräte dieser Schleife hat und diese Steuer- und Kontrollvorrichtung sich am Knotenpunkt zwischen dieser Nebenschleife und der Hauptschleife (10) befindet, wobei jedes Gerät mit einer Kommunikationsvorrichtung (14) versehen ist, die einen Datenverarbeitungsschaltkreis (16) für das Gerät und einen Schaltkreis (15) umfaßt, der für die Übertragungen der Meldungen vorbehalten ist.

2. Anlage nach Anspruch 1, dadurch gekennzeichnet, daß ein allgemeines Steuerpult (34) in einer der Schleifen (11) angeordnet ist.

3. Anlage nach Anspruch 1 oder 2, dadurch gekennzeichnet, daß in einer ersten Nebenschleife (11) Positionssteuerungen (31, 32) der Geräte des Röntgensaals angeordnet sind, nämlich des Patienten-Tragetisches und des Trägers der Einheit Röntgenröhre-Detektor(en).

4. Anlage nach Anspruch 3, dadurch gekennzeichnet, daß sich in der ersten Nebenschleife (11), in Serie mit den Positionssteuerungen, die Steuerung (30) für die Verteilung der elektrischen Energie an die Geräte des Röntgensaals und ein Positionssteuerpult (33) befinden.

5. Anlage nach Anspruch 2 und 3, dadurch gekennzeichnet, daß das allgemeine Steuerpult (34) der Anlage in Serie mit den Positionssteuerungen (31, 32) der Geräte in der ersten Nebenschleife (11) geschaltet ist.

6. Anlage nach einem der Ansprüche 1 bis 5, dadurch gekennzeichnet, daß die Steuerungen der Bilderzeugungsgeräte: Photoapparat (38) und Videokamera (37) in Serie in einer zweiten Nebenschleife (12) angeordnet sind.

7. Anlage nach Anspruch 6, dadurch gekennzeichnet, daß wenigstens die Steuerung (35, 39, 40) eines der folgenden Elemente ebenfalls in Serie in der zweiten Schleife (12) angeordnet ist: ein Kollimator, ein Filmwechsler und ein Bildverstärker.

8. Anlage nach einem der Ansprüche 1 und 6, dadurch gekennzeichnet, daß die Steuerung (4) eines automatischen Kontrastprodukt-Injektionsgeräts durch eine Steuer- und Kontrollvorrichtung (27) am Knoten zwischen der Hauptschleife (10) und der zweiten Nebenschleife (12) erfolgt.

9. Anlage nach einem der Ansprüche 1 bis 8, dadurch gekennzeichnet, daß sich in der Hauptschleife (10) eine Vorrichtung (21) zur Kontrolle der Röntgenstrahl-Erzeugungsmittel befindet.

10. Anlage nach einem der Ansprüche 1 bis 9, dadurch gekennzeichnet, daß sich in der Hauptschleife (10) ein Rechengerät (28) befindet, um eine digitale Bearbeitung eines aufgezeichneten Bildes durchzuführen.

11. Anlage nach Anspruch 1, dadurch gekennzeichnet, daß der Meldungsübertragungskreis (15) einen einfachen Schalter (54) hat, der im Falle einer Störung des entsprechenden Geräts geschlossen wird, um die Schleife, in der sich dieses Gerät befindet, geschlossen zu halten.

12. Anlage nach einem der vorausgehenden Ansprüche, dadurch gekennzeichnet, daß die Informationen über Lichtleitfasern (52) übermittelt werden, wobei jeder Übertragungskreis (15) an seinem Eingang einen Wandler (51) für die Umwandlung von Leuchtsignalen in elektrische Signale umfaßt, und an seinem Ausgang einen Wandler (53) für die Umwandlung von elektrischen Signalen in Leuchtsignale.

13. Anlage nach einem der vorausgehenden Ansprüche, dadurch gekennzeichnet, daß jedem Gerät eine Kommunikations- und Verarbeitungsvorrichtung beigegeben ist, wobei die verschiedenen Kommunikations- und Verarbeitungsvorrichtungen alle identisch sind.

14. Anlage nach einem der vorausgehenden Ansprüche, dadurch gekennzeichnet, daß sie ein zentrales Steuerungsorgan umfaßt, das bei jeder Einschaltung der Anlage einerseits die Konfiguration der letzteren bestimmt, das heißt: die Geräte der Anlage und die Schleife, in der sich jedes Gerät befindet, identifiziert, und andererseits diese Konfigurationsinformation gespeichert hält, wobei die Bestimmung mittels zusätzlichen Steuerungsorganen an den Anschlüssen zwischen benachbarten Schleifen durchgeführt wird.

15. Anlage nach Anspruch 14, dadurch gekennzeichnet, daß für bestimmte Operationen ein Steuerungsorgan das Zentralorgan bildet, wohingegen für andere Operationen ein anderes Organ dieses Zentralorgan bildet.

## Claims

1. A radiology installation comprising a group of devices between which information signals are able to circulate, characterized in that for the communication of the signals the installation comprises at least two loops (10, 11 and 12) between which the information is able to circulate, each loop being capable of communicating information which does not pass through another loop, each loop connecting different devices of the installation, said installation comprising a principal loop (10) and secondary or specialized loops (11 and 12), each secondary loop comprising an actuating and control device (20 and 27) for the group of devices of this loop, said actuating and control device being located at the link being this secondary loop and the principal loop (10), each device being acted upon by a device (14) for transfer comprising a circuit (16) for the processing of information for the device and a circuit (15) reserved for the communication of messages.

2. The installation as claimed in claim 1, characterized in that a master control console (34) is disposed in one of the loops (11).

3. The installation as claimed in claim 1 or claim 2, characterized in that the position controls (31 and 32), that is to say the patient bearing table and the support for the group of comprising an X-ray tube and the detector or detectors, are placed in a first secondary loop.

4. The installation as claimed in claim 3, characterized in that the first secondary loop (11), in series with the position controls, includes within it the electrical power distribution control (30) for the devices in the radiology room and a console (33) for position control.

5. The installation as claimed in claim 2 and claim 3, characterized in that the master control console (34) for the installation is in series with the position controls (31 and 32) of the devices in the first secondary loop (11).

6. The installation as claimed in any one of the claims 1 through 5, characterized in that the controls for the image forming devices: i.e. the photographic device (38) and the video camera (37), are placed in series in a second secondary loop (12).

7. The installation as claimed in claim 6, characterized in that at least the control (35, 39 and 40) and one of the following elements is also placed in series in the second loop (12): i.e. a collimator, a film changer and an image intensifier.

8. The installation as claimed in claims 1 and 6, characterized in that the control (4) for an automatic device for the injection of a contrast agent is performed by the device (27) for the actuation and control at the link between the principal loop (10) and the second secondary loop (12).

9. The installation as claimed in any one of the claims 1 through 8, characterized in that the principal loop (10) includes within it a device (21) for the actuation of the X-ray generators.

10. The installation as claimed in any one of the claims 1 through 9, characterized in that the principal loop (10) includes within it a computing device (28) in order to perform numerical processing of the recorded image.

11. The installation as claimed in claim 1, characterized in that the circuit (11) for the communication of messages comprises a simple switch (54) which is closed in the case of malfunction of the corresponding device in order to keep the loop closed in which such device is included.

12. The installation as claimed in any one of the preceding claims, characterized in that the information is communicated by the intermediary of optic fibers (52), each communication circuit (15) having at its input a converter (51) for the conversion of the light signal into an electrical signal and at its output a converter (53) for the conversion of the electrical signal into a light signal.

13. The installation as claimed in any one of the preceding claims, characterized in that each device has associated with it a transfer and processing device, the various devices for transfer and processing being mutually identical.

14. The installation as claimed in any one of the preceding claims, characterized in that it comprises a central controlling means which, every time the installation is supplied with power, on the one hand ascertains the configuration of the installation, that is to say it identifies the devices included in the installation and the loop in which each of same is included and on the other hand stores such configuration information in a memory, this ascertainment being performed by the intermediary of control means linked with the connections between adjacent loops.

15. The installation as claimed in claim 14, characterized in that for certain operations a controlling means constitutes the central means, whereas for other operations, another means constitutes the central means.

FIG_1

# FIG_2

# FIG_3

# FIG_4